(19) Europäisches Patentamt
European Patent Office
Office européen des brevets

(11) Numéro de publication: **0 300 518 B1**

(12) # FASCICULE DE BREVET EUROPEEN

(45) Date de publication de fascicule du brevet: **02.09.92**

(51) Int. Cl.[5]: **C07F 5/02**, C07C 277/00, C07C 279/00

(21) Numéro de dépôt: **88201153.9**

(22) Date de dépôt: **07.06.88**

(54) **Composés guanidiniques comprenant un ion tétraphénylborate, procédé d'obtention de ces composés et utilisation des composés lors de la synthèse peptidique.**

(30) Priorité: **19.06.87 FR 8708695**

(43) Date de publication de la demande:
**25.01.89 Bulletin 89/04**

(45) Mention de la délivrance du brevet:
**02.09.92 Bulletin 92/36**

(84) Etats contractants désignés:
**AT BE CH DE ES FR GB GR IT LI LU NL SE**

(56) Documents cités:

**CHEMICAL ABSTRACTS, vol. 95, no. 4, 27 juillet 1981, page 400, résumé no. 31163w, Columbus, Ohio, US; O. POPOVYCH: "Solubility data. Guanidine tetraphenylborate-water system", & SOLUBILITY DATA SER. 1981, 18, 91**

(73) Titulaire: **SOLVAY**
**Rue du Prince Albert, 33**
**B-1050 Bruxelles(BE)**

(72) Inventeur: **Callens, Roland**
**Flonkedreef 3**
**B-9810 Gent-Drongen(BE)**
Inventeur: **Collin, André**
**Rue du Pont Piraux, 3A**
**B-6338 Ligny(BE)**

(74) Mandataire: **Lechien, Monique et al**
**Solvay Département de la Propriété Industrielle Rue de Ransbeek, 310**
**B-1120 Bruxelles(BE)**

EP 0 300 518 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne des composés guanidiniques comprenant un ion tétraphénylborate associé à un produit comprenant une fonction guanidinique ainsi qu'un procédé d'obtention de tels composés qui peuvent être utilisés comme moyen pour solubiliser le produit dont la protection de la fonction guanidinique est assurée, notamment lors de la synthèse peptidique à partir d'acides aminés ou de peptides.

La demande de brevet allemand DOS 2716477 divulgue notamment des sels de guanidine N,N′,N″ substitués de formule générale :

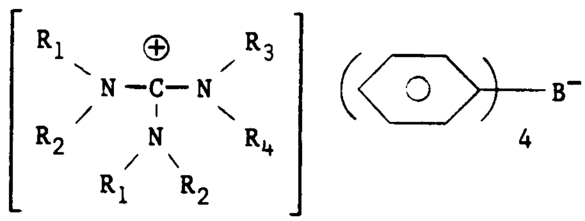

dans laquelle $R_1$, $R_2$, ,$R_3$ et $R_4$ représentent un radical aliphatique, cyclique, aliphatique arylé, aromatique ou hétérocyclique et dans laquelle, seul $R_1$ peut être un atome d'hydrogène.

Ces produits, protonés sur l'atome de carbone du groupement guanidinique substitué, sont synthétisés à partir d'un dérivé halogéné d'acide carbamique et de thiourée substituée et peuvent être utilisés comme catalyseurs, agents de protection des plantes et teintures pharmaceutiques.

La présente invention vise à fournir une nouvelle catégorie de composés dont la formule de structure est voisine de celle des produits précités mais dont toutes les valences des atomes d'azote de la fonction guanidinique à l'exception d'une seule sont saturées par des atomes d'hydrogène.

Les composés selon l'invention comportent une fonction guanidinique et un ion tétraphénylborate et répondent à la formule générale :

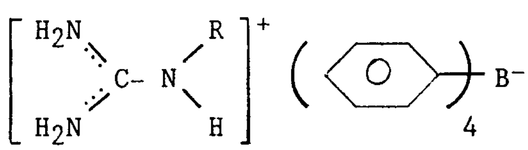

dans laquelle R représente un radical organique de formule générale :

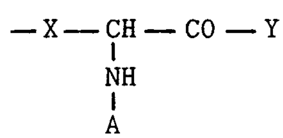

dans laquelle X, A et Y représentent indépendamment les uns des autres des radicaux aliphatiques linéaires, ramifiés ou cycliques, substitués ou non, saturés ou non, des radicaux aromatiques, des radicaux aliphatiques arylés ou des radicaux hétérocycliques. A peut en outre également représenter un atome d'hydrogène et Y un groupement hydroxyle ou un atome d'halogène. Généralement,

- X représente un radical aliphatique linéaire, ramifié ou cyclique, non substitué, saturé ou non, contenant jusqu'à 25 atomes de carbone,
- A représente un atome d'hydrogène, un radical aliphatique ou aromatique comportant des hétéroatomes ou non, tels que le benzyloxycarbonyle ou le tert-butyloxycarbonyle agissant comme groupement protecteur ou le N-hydroxysuccinimide agissant comme groupement d'activation ; un ou plusieurs acides aminés liés par des liaisons peptidiques, dont certaines fonctions sont éventuellement substituées par le benzyloxycarbonyle ou le tert-butyloxycarbonyle agissant comme groupement protecteur ou le N-hydroxysuccinimide agissant comme groupement d'activation, un groupement protecteur tel que le benzyloxycarbonyle ou le ter-butyloxycarbonyle,
- Y représente un groupement hydroxyle ; un atome d'halogène ; un radical aliphatique ou aromatique, comportant éventuellement des hétéroatomes, tels que le benzyloxycarbonyle ou le tert-butyloxycarbonyle agissant comme groupement protecteur ou le N-hydrosuccinimide agissant comme groupement d'activation ; un groupement aminé ; un acide aminé ou un peptide dont certaines fonctions sont éventuellement substituées par des groupements protecteurs ou des groupements d'activation ainsi que par des groupements aminés de formule générale $NR_1R_2$ dans laquelle $R_1$ et $R_2$ représentent indépendamment l'un de l'autre un atome d'hydrogène ou un groupement alkyle comptant de 1 à 3 atomes de carbone.

De préférence :

- X représente un radical alkyle de formule générale $\leftarrow CH_2 \rightarrow_n$ dans laquelle n est un nombre entier compris entre 1 et 10.
- A repésente un atome d'hydrogène, un acide aminé, un peptide ou un groupement protecteur.
- Y représente un groupement hydroxyle, un groupement protecteur, un groupement d'activation, un acide aminé ou un peptide.

De manière tout particulièrement préférée :

- X représente le radical $\leftarrow CH_2 \rightarrow_n$ dans lequel n est compris entre 1 et 6.
- A représente un atome d'hydrogène, un groupement protecteur tel que notamment le benzyloxycarbonyle (Z) ou le tert-butyloxycarbonyle (t-Boc), un acide aminé ou un peptide éventuellement substitués par ces mêmes groupements protecteurs.
- Y représente un groupement hydroxyle, un groupement protecteur tel qu'un benzylester, un groupement d'activation tel que le N-hydroxysuccinimide, un acide aminé ou un peptide éventuellement substitués par des groupements protecteurs et/ou d'activation ou un groupement aminé.

Enfin, de bons résultats ont été obtenus lorsque :

- X représente le radical $\leftarrow CH_2 \rightarrow_3$.
- A représente un atome d'hydrogène, un groupement protecteur tel que le benzyloxycarbonyle ou le tert-butyloxycarbonyle ou un acide aminé éventuellement substitué par le benzyloxycarbonyle, le tert-butyloxycarbonyle ou le N-hydroxysuccinimide.
- Y représente un groupement hydroxyle ou un acide aminé éventuellement substitué par le benzyloxycarbonyle, le tert-butyloxycarbonyle ou le N-hydroxysuccinimide ou un groupement aminé.

Par acide aminé, on entend tout acide organique possédant au moins une fonction carboxylique et au moins une fonction amine primaire ou secondaire tel que les acides aminés naturels connus ou les acides aminés synthétiques. Par peptide, on entend tout peptide issu de toute association d'acides aminés naturels ou synthétiques.

Par groupement protecteur, on entend tout composé cité à cet effet dans la littérature et plus particulièrement par :

- M. BODANSZKY, Principles of Peptide Synthesis, 1984, Volume 16, Reactivity and Structure Concepts in Organic Chemistry,
- M. BODANSZKY, A. BODANSZKY, The Practice of Peptide Synthesis, 1984, Volume 21, Reactivity and Structure Concepts in Organic Chemistry.

A titre illustratif, les groupements protecteurs suivants peuvent être mis en oeuvre dans les composés de l'invention :

- des groupements protecteurs type acyle tels que notamment formyle, trifluoroacétyle, phthaloyle, 4-toluènesulfonyle, benzènesulfonyle, 2-nitrophénylsulfényle,
- des groupements protecteurs type uréthane aromatique tels que notamment le benzyloxycarbonyle substitué ou non tel que le p-chlorobenzyloxycarbonyle, le p-nitrobenzyloxycarbonyle, p-bromobenzyloxycarbonyle, p-méthoxybenzyloxycarbonyle, benzhydroxyloxycarbonyle, le 2-(4-biphénylyl)propyl(2)-oxycarbonyle, le 2-(3,5-diméthyloxyphényl)propyl(2)oxycarbonyle, le triphénylphosphonoéthyloxycarbonyle,
- des groupements protecteurs type uréthane aliphatique tels que notamment le tert-butyloxycarbonyle, le diisopropylméthoxycarbonyle, l'isopropyloxycarbonyle, l'éthoxycarbonyle, l'allyloxycarbonyle, le 9-fluorenylméthyloxycarbonyle, le 2-méthylsulfonyléthyloxycarbonyle, le 2,2,2-trichloroéthyloxycarbonyle,
- des groupements protecteurs type uréthane cycloalkyle tels que notamment le cyclopentyloxycarbonyle, l'adamantyloxycarbonyle, le cyclohexyloxycarbonyle, le tert-amyloxycarbonyle, l'isobornyloxycarbonyle,
- des groupements protecteurs type thiouréthane tels que notamment le phénylthiocarbonyle,
- des groupements protecteurs type alkyle tels que notamment le triphénylméthyle(trityle) et le benzyle,
- des groupements trialkylsilane tel que le triméthylsilane,
- des groupements alkoxy tel que notamment l'ester de méthyle, l'ester d'éthyle, le tert-butyl ester, le benzylester.

Par groupement d'activation, on entend tout groupement d'activation connu ou non, tel que ceux cités dans la littérature et plus particulièrement dans les articles de :

- M. BODANSZKY,
- M. BODANSZKY, A. BODANSZKY cités ci-avant.

Habituellement, on met en oeuvre comme groupement d'activation un groupement oxycarbonyle, oxycarboxyle, N-oxyimidoyle, imidazoyle tel que notamment le pivaloyle oxycarbonyle, le N-hydroxysuccinimidoyle, le dicyclohexylcarbodiimidoyle, le 4-nitrophénylester.

Les composés préférés selon l'invention répondent aux formules :

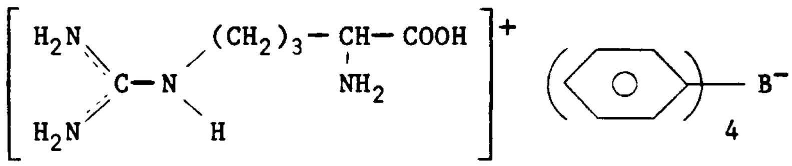

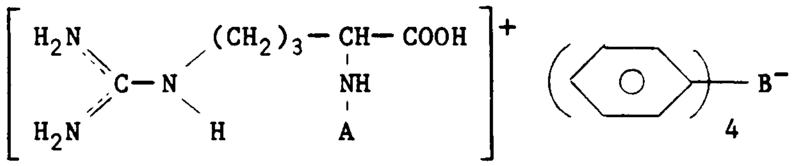

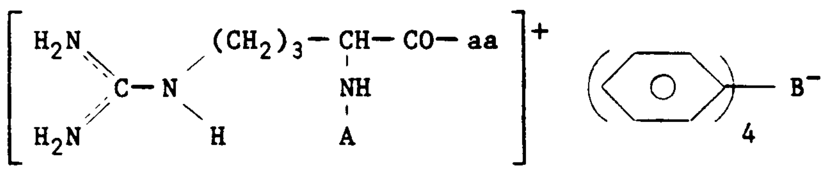

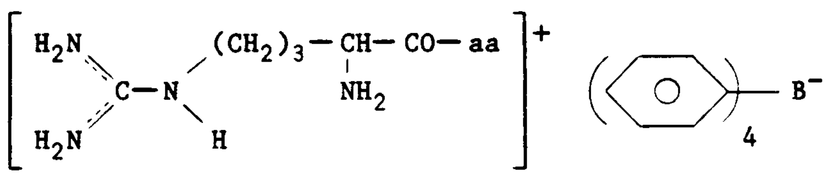

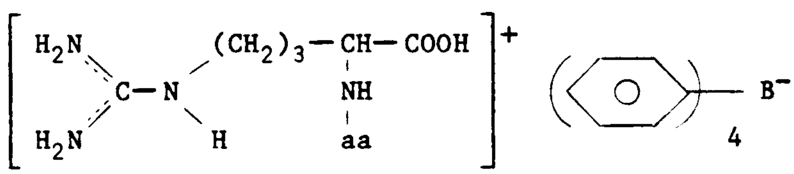

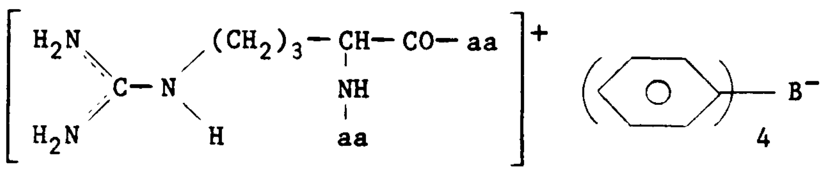

dans lesquelles aa représente un acide aminé ou un peptide lié par une liaison peptidique à l'arginine et dont les fonctions amines et carboxyliques sont éventuellement protégées ou substituées. Une telle protection est rendue nécessaire lorsque certains groupements fonctionnels, en particulier amine ou carboxylique, doivent être bloqués pour éviter qu'ils interviennent dans des réactions ultérieures lors de la mise en oeuvre du composé. La fonction carboxylique terminale de certains acides aminés ou peptides peut en outre être substituée par un groupement aminé tel que le groupement $-NH_2$ ou $-NH-CH_2-CH_3$.

De manière particulièrement préférée, aa représente un acide aminé.

Les composés selon l'invention peuvent être préparés par toute synthèse organique appropriée

regroupant des réactions connues ou non et s'appliquant de façon générale ou de façon particulière à un seul composé déterminé ou à une famille de composés.

Un procédé ayant donné de bons résultats pour la préparation des composés selon l'invention consiste à mettre en oeuvre un sel de tétraphénylborate et un produit comprenant un groupement guanidinique.

Les sels de tétraphénylborate mis en oeuvre pour la synthèse des composés selon l'invention peuvent être formés à partir de n'importe quelle base inorganique ou organique.

Habituellement, on utilise une base organique et plus particulièrement une base organique azotée telle q'une amine secondaire, tertiaire ou hétérocyclique. De bons résultats ont été obtenus avec la triéthylamine, la diisopropyléthylamine, la N-méthylmorpholine, la N-éthylmorpholine, la N-méthylpiperidine, la N-éthylpiperidine, la tri- n butylamine, la dicyclohexylamine et l'imidazole.

Le sel de tétraphénylborate est mis en oeuvre dans la réaction en présence d'un solvant ou d'un mélange de solvants. Généralement, on met en oeuvre un seul solvant organique, polaire, tel que notamment le diméthylsulfoxide (DMSO), le N,N-diméthylformamide (DMF), la N-méthylpyrrolidone, l'acétonitrile. Un solvant qui a donné de bons résultats est le N,N-diméthylformamide.

La quantité de sel de tétraphénylborate mis en oeuvre, peut varier dans de larges limites. En général, on met en oeuvre de 20 à 1 mole de sel de tétraphénylborate par mole de produit comprenant la fonction guanidinique. De préférence, on met en oeuvre de 10 à 1 mole de sel de tétraphénylborate. De manière particulièrement préférée, 1 mole de sel de tétraphénylborate est mise en oeuvre par mole de produit comprenant la fonction guanidinique.

Les autres conditions opératoires utilisées dans le procédé pour la préparation des composés selon l'invention ne sont pas critiques pour l'invention. Ainsi, la pression à laquelle est effectué le procédé, est généralement comprise entre 0,1 et 10 bar et de bons résultats ont été obtenus à la pression atmosphérique. La température à laquelle est effectué le procédé, est habituellement comprise entre -60 et 100 ° C et peut varier selon la nature des réactifs et le composé que l'on veut finalement préparer.

Le procédé peut être réalisé dans tout appareillage conçu à cet effet.

Les composés de l'invention sont utilisables comme intermédiaires de synthèse chimique.

Du fait de leur solubilité dans les solvants organiques, ils peuvent notamment être mis en oeuvre lors de leur propre couplage avec d'autres produits; en particulier lors de la synthèse de peptides à partir d'acides aminés telle que notamment décrit dans la demande de brevet européen 0184243 qui se rapporte à un procédé faisant intervenir un trialkylcyanosilane.

En outre, le fait que l'ion tétraphénylborate est le contre-ion de la fonction guanidinique, dont il assure la protection, permet d'assurer la protection de cette fonction guanidinique lors de la réalisation de couplages sélectifs et d'obtenir ainsi des produits d'une plus grande pureté lors des synthèses. En effet, à la fin de la synthèse peptidique, l'ion tétraphénylborate est facilement déplacé du produit comprenant la fonction guanidinique par toute méthode connue, par exemple par addition d'eau, ce qui permet de libérer d'une part la fonction guanidinique et d'autre part de former à nouveau le sel de tétraphénylborate de départ. Ce sel peut ainsi être récupéré et recyclé lorsque le milieu de réaction n'est pas acide.

Plus particulièrement, l'invention concerne l'utilisation de l'ion tétraphénylborate comme moyen pour solubiliser dans des solvants organiques l'arginine et les peptides contenant des arginines libres mais protonées dans la chaîne latérale.

Les exemples qui suivent servent à illustrer l'invention.

Dans ces exemples, les abréviations suivantes ont été utilisées :

- Arg : arginine
- Gly : glycine
- Leu : leucine
- Pro : proline
- O-Piv : pivaloyloxy(triméthyl acétyloxy)
- O-Succ : N-hydroxysuccinimide
- Z : groupement protecteur de type benzyloxycarbonyle
- t-Boc : groupement protecteur de type tert-butyloxycarbonyle
- DMF : N,N-diméthylformamide
- Et : groupement éthyle $-CH_2-CH_3$.

Exemple 1 : Synthèse du composé formé d'arginine et de tétraphénylborate

Dans un réacteur thermostatisé de 250 ml équipé d'un condenseur et d'un système d'agitation, on introduit successivement 8,71 g (0.050 mole) d'arginine protonée (H-Arg OH), 23,18 g (0,055 mole) de tétraphénylborate de triéthylammonium, puis 100 ml de N,N-diméthylformamide (DMF).

Le mélange est maintenu à 25 ° C à pression atmosphérique sous agitation vigoureuse durant 5 minutes.

On obtient une concentration de 0,397 mole par kg en composé formé d'arginine et de tétraphénylborate.

Ce mélange peut être utilisé directement pour la synthèse du peptide t-Boc-Leu-Arg OH.

Exemple 2 : Utilisation du composé formé d'arginine et de tétraphénylborate lors de la synthèse du peptide t-Boc-Leu-Arg OH

a) Synthèse du peptide t-Boc-Leu-Arg OH

Au mélange, obtenu à l'exemple 1, et maintenu à 25 ° C, on ajoute sous forme solide 17,57 g (0,054 mol) de leucine (Leu) dont la fonction amine est protégée par le groupement tert-butyloxycarbonyle (t-Boc) et dont la fonction carboxylique est activée par du N-hydroxysuccinimide.

Après 4 heures à 25 ° C, la solution réactionnelle contient le dipeptide t-Boc-Leu-Arg OH obtenu avec un taux de couplage voisin de 100 %.

b) Séparation du tétraphénylborate de triéthylammonium

Dans un réacteur thermostatisé de 2 l équipé d'un condenseur, d'une ampoule d'introduction et d'un système d'agitation, on introduit 900 ml d'eau. La température est amenée à 5 ° C.

Par l'intermédiaire de l'ampoule d'introduction, la solution réactionnelle contenant le dipeptide t-Boc-Leu-Arg OH est ajoutée en 30 minutes sous agitation vigoureuse en maintenant la température entre 5 et 10 ° C.

Le tétraphénylborate de triéthylammonium précipite progressivement en 1 heure à 5 ° C. Le précipité est séparé par filtration sur plaque frittée de porosité 4 et est lavé avec 4 fois 50 ml d'eau.

Après séchage à 50 ° C sous pression réduite à 1 mb, on récupère 22,95 g de tétraphénylborate de triéthylammonium, soit 99 % de la quantité mise en oeuvre.

c) Extraction et purification du peptide t-Boc-Leu-Arg OH

Le filtrat et les eaux de lavage contenant t-Boc-Leu-Arg OH sont concentrés dans un évaporateur rotatif à 60 ° C et sous une pression réduite à 1 mb jusqu'à l'obtention d'un résidu d'environ 20 g.

Le résidu est traité à température ambiante dans un réacteur équipé d'un système d'agitation par 400 ml d'acétone. Le résidu se désagrège lentement et donne naissance à un précipité blanc.

Après 2 heures de traitement sous agitation vigoureuse, la suspension obtenue est conservée à 5 ° C durant 24 heures.

Le précipité formé est ensuite séparé par filtration sur plaque frittée de porosité 4 et est lavé avec 4 fois 20 ml d'acétone.

Après séchage à 40 ° C sous pression réduite à 1 mb, on récupère 17,5 g d'un solide blanc pulvérulent dont le contenu en dipeptide t-Boc-Leu-Arg est de 95 %, soit un rendement en dipeptide pur de 86 %.

Exemple 3 : Synthèse du composé formé de tétraphénylborate et d'arginine protégée par un groupement benzyloxycarbonyle

Dans un réacteur thermostatisé de 2 l équipé d'un condenseur et d'un système d'agitation, on introduit successivement 1500 ml de DMF, 154 g (0,5 mole) d'arginine dont la fonction $\alpha$-aminée est protégée par un groupement benzyloxycarbonyle (Z-Arg) et 210,5 g (0,5 mole) de tétraphénylborate de triéthylammonium.

Le mélange est chauffé à 40 ° C pendant 1 heure à pression atmosphérique sous agitation vigoureuse. La solution obtenue est ensuite refroidie à -20 ° C et est stockée à cette température.

On obtient une concentration de 0,280 mole par kg en composé formé de tétraphénylborate et d'arginine dont la fonction $\alpha$-aminée est protégée par un groupement benzyloxycarbonyle.

Exemple 4 : - Synthèse du dipeptide Z-Arg-Pro
- Utilisation du composé obtenu à l'exemple 3

a) Synthèse du dipeptide Z-Arg-Pro

Dans un réacteur (I) thermostatisé de 250 ml équipé d'un condenseur, d'un système d'agitation et d'un dispositif permettant de maintenir une couverture gazeuse d'azote et raccordé à un scrubber à hydroxyde de sodium, on introduit successivement 69 g (0,6 mol) de proline et 119 g (1,2 mol) de triméthylcyanosilane.

Le mélange est chauffé à 40°C pendant 5 minutes sous agitation. La solution obtenue (sol I) est homogène et est stockée à température ambiante.

Dans un autre réacteur (II) thermostatisé de 3 l équipé d'un condenseur, d'un système d'agitation, d'un dispositif permettant de maintenir une couverture gazeuse d'azote et raccordé à un scrubber à hydroxyde de sodium et d'une ampouler d'introduction, on introduit successivement 200 ml de DMF, 67 g (0,55 mol) de chlorure de pivaloyle (Piv-Cl) et 44 g (0,55 mol) de pyridine.

On amène cette solution à -30°C.

Ensuite, on introduit la solution obtenue lors de l'exemple 3 sous agitation vigoureuse par l'intermédiaire de l'ampoule d'introduction en 10 minutes en maintenant la température du milieu réactionnel entre -30 et -15°C.

Ce milieu réactionnel est maintenu à -15°C durant 5 minutes.

Puis, on introduit la solution (sol I) en 5 minutes. La solution réactionnelle est maintenue sous agitation à -15°C durant 1 heure, puis à 0°C durant 2 heures.

Le taux de couplage est voisin de 85 %.

b) Séparation du sel de tétraphénylborate de triéthylammonium

Dans un réacteur (III) thermostatisé de 10 l présentant le même équipement que le réacteur (II), on introduit 5 l d'eau refroidie à 5°C, puis en 60 minutes la solution réactionnelle.

Le mélange est maintenu à 5°C sous bonne agitation, le sel de tétraphénylborate de triéthylammonium précipite progressivement.

Après 1 heure, le précipité est séparé par filtration sur plaque frittée de porosité 4 et est lavé avec 4 fois 250 ml d'eau.

Après séchage à 50°C sous pression réduite à 1 mb, on récupère 209 g de sel de tétraphénylborate de triéthylammonium, soit plus de 99 % de la quantité mise en oeuvre.

c) Extraction et purification du dipeptide

Le filtrat et les eaux de lavage sont concentrés dans un évaporateur rotatif à 40°C sous une pression réduite de 1 mb jusqu'à l'obtention d'un résidu d'environ 300 g.

Ce résidu est dissous dans 1 l d'eau, le pH est ajusté à 7,5 à température ambiante par addition progressive de triéthylamine jusqu'à la stabilisation.

Le dipeptide Z-Arg-Pro précipite progressivement sous forme de zwitterion.

Après 2 heures, le précipité est séparé par filtration sur plaque frittée de porosité 4.

Le précipité est lavé avec 4 fois 20 ml d'eau puis séché sous une pression réduite à 1 mb.

On récupère ainsi 130 g d'un solide blanc pulvérulent dont le contenu en dipeptide Z-Arg-Pro est voisin de 100 %, soit un rendement en produit pur de 85 %.

Exemple 5 : Synthèse du composé formé de tétraphénylborate et du dipeptide protégé Z-Arg-Pro

Dans un réacteur de 500 ml équipé d'un condenseur et d'un système d'agitation, on introduit successivement 200 ml de DMF, 20,3 g (0,050 mole) du dipeptide protégé Z-Arg-Pro tel qu'obtenu dans l'exemple 4, 19,4 g (0,050 mole) de tétraphénylborate d'imidazolidinium.

La solution devient limpide après 1 heure d'agitation à température ambiante et sous pression atmosphérique.

On obtient une concentration de 0,220 mole par kg du composé formé de tétraphénylborate et du dipeptide protégé Z-Arg-Pro.

Exemple 6 : - Synthèse de Z-Arg-Pro-NH-Et
- Utilisation du composé obtenu à l'exemple 5

A la solution obtenue à l'exemple 5, on ajoute 10,1 g (0,063 mol) de carbonyldiimidazole dissous dans 60 ml de DMF. Le mélange réactionnel est placé sous agitation à température ambiante durant 90 minutes.

On ajoute ensuite 12,2 g (0,150 mol) de chlorhydrate d'éthylamine dissous dans 100 ml de DMF. La

solution réactionnelle est placée 15 heures sous agitation à température ambiante, la réaction est terminée alors.

Ensuite, la solution réactionnelle est concentrée dans un évaporateur rotatif à 50 ° C sous une pression réduite de 1 mb jusqu'à l'obtention d'un résidu d'environ 100 g.

Le résidu est repris par 800 ml d'eau. On effectue sur la solution obtenue deux extractions par un mélange de 350 $cm^3$ d'acétate d'éthyle et 150 $cm^3$ d'éther éthylique. Les phases organiques séparées par décantation sont évaporées à sec dans un évaporateur rotatif à 40 ° C sous pression réduite de 1 mb.

Un résidu de 34,3 g est récupéré. Il est essentiellement constitué du composé formé du peptide Z-Arg-Pro-NH-Et et de tétraphénylborate, le rendement de couplage est donc de plus de 90 %.

L'échange de l'anion tétraphénylborate en anion acétate peut être réalisé par traitement du peptide en solution dans un mélange eau-méthanol (proportion 1/2) sur une résine anionique du type Bio Rad AG 1x8 forme acétate.

Exemple 7 : Synthèse du composé formé de tétraphénylborate et du peptide t-Boc-Leu-Arg-Pro

Dans un réacteur de 1 l équipé d'un condenseur et d'un système d'agitation, on introduit successivement 300 ml de DMF, 50,8 g (0,105 mole) du peptide t-Boc-Leu-Arg-Pro et 40,6 g (0,105 mole) de tétraphénylborate d'imidazolidinium.

Le mélange est agité 10 minutes à température ambiante et à pression atmosphérique. Une solution homogène est alors obtenue.

Elle contient une concentration de 0,280 mole par kg de composé formé de tétraphénylborate et du peptide t-Boc-Leu-Arg-Pro.

Exemple 8 : - Synthèse du peptide t-Boc-Leu-Arg-Pro-Gly $NH_2$
- Utilisation du composé obtenu à l'exemple 7

A la solution homogène obtenue à l'exemple 7, on ajoute 19,5 g (0,120 mol) de carbonyldiimidazole dissous dans 100 ml de DMF.

Le mélange est agité 100 minutes à température ambiante.

A celui-ci, on ajoute 82,8 g (0,210 mol) de sel de tétraphénylborate de glycine amide protonée dissous dans 200 ml de DMF. Cette solution réactionnelle est agitée 15 heures à température ambiante.

Dans un réacteur thermostatisé de 5 l équipé d'un condenseur, d'un système d'agitation et d'une ampoule d'introduction, on introduit 57,8 g (0,420 mol) de chlorhydrate de triéthylamine dissous dans 3 l d'eau.

Par l'intermédiaire de l'ampoule d'introduction, on ajoute la solution réactionnelle en 30 minutes sous agitation vigoureuse en maintenant la température entre 5 et 10 ° C.

Le tétraphénylborate de triéthylammonium précipite progressivement.

Après 1 heure à 5 ° C, le précipité est séparé par filtration sur plaque frittée de porosité 4 et est lavé avec 4 fois 30 ml d'eau.

Après séchage à 50 ° C sous pression réduite à 1 mb, on récupère 131,3 g de tétraphénylborate de triéthylammonium correspondant à 99 % de la quantité d'ion de tétraphénylborate mise en oeuvre.

Le filtrat et les eaux de lavage sont concentrés dans un évaporateur rotatif à 50 ° C sous une pression réduite à 1 mb jusqu'à l'obtention d'un résidu de 200 g.

Ce résidu contient 49,4 g (0,091 mol) de peptide t-Boc-Leu-Arg-Pro-Gly-$NH_2$ soit un rendement de couplage de 87 %.

**Revendications**
**Revendications pour les Etats contractants suivants : AT, BE, CH, DE, FR, GB, IT, LI, LU, NL, SE**

1. Composés guanidiniques comprenant un ion tétraphénylborate caractérisés en ce qu'ils répondent à la formule générale :

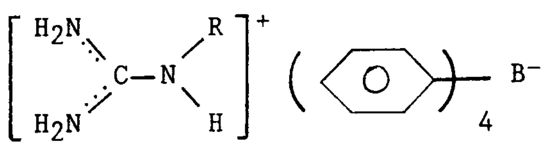

dans laquelle R représente un radical organique de formule générale :

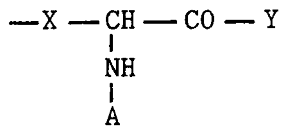

dans laquelle :

- X représente un radical aliphatique linéaire, ramifié ou cyclique, non substitué, saturé ou non, contenant jusqu'à 25 atomes de carbone,
- A représente un atome d'hydrogène, un radical aliphatique ou aromatique comportant des hétéroatomes ou non, tels que le benzyloxycarbonyle ou le tert-butyloxycarbonyle agissant comme groupement protecteur ou le N-hydroxysuccinimide agissant comme groupement d'activation ; un ou plusieurs acides aminés liés par des liaisons peptidiques, dont certaines fonctions sont éventuellement substituées par le benzyloxycarbonyle ou le tert-butyloxycarbonyle agissant comme groupement protecteur ou le N-hydroxysuccinimide agissant comme groupement d'activation, un groupement protecteur tel que le benzyloxycarbonyle ou le ter-butyloxycarbonyle,
- Y représente un groupement hydroxyle ; un atome d'halogène ; un radical aliphatique ou aromatique, comportant éventuellement des hétéroatomes, tels que le benzyloxycarbonyle ou le tert-butyloxycarbonyle agissant comme groupement protecteur ou le N-hydrosuccinimide agissant comme groupement d'activation ; un groupement aminé ; un acide aminé ou un peptide dont certaines fonctions sont éventuellement substituées par des groupements protecteurs ou des groupements d'activation ainsi que par des groupements aminés de formule générale $NR_1R_2$ dans laquelle $R_1$ et $R_2$ représentent indépendamment l'un de l'autre un atome d'hydrogène ou un groupement alkyle comptant de 1 à 3 atomes de carbone.

**2.** Composés selon la revendication 1 caractérisés en ce que :

- X représente le radical $-(CH_2)_3-$ ;
- A représente un atome d'hydrogène, un groupement protecteur tel que le benzyloxycarbonyle ou le tert-butyloxycarbonyle ou un acide aminé éventuellement substitué par le benzyloxycarbonyle, le tert-butyloxycarbonyle ou le N-hydroxysuccinimide;
- Y représente un groupement hydroxyle ou un acide aminé éventuellement substitué par le benzyloxycarbonyle, le tert-butyloxycarbonyle ou le N-hydroxysuccinimide ou un groupement aminé.

**3.** Composés selon la revendication 1 ou 2 caractérisés en ce qu'ils répondent aux formules :

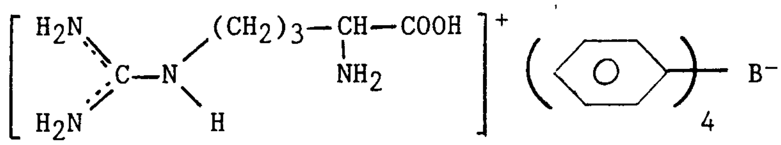

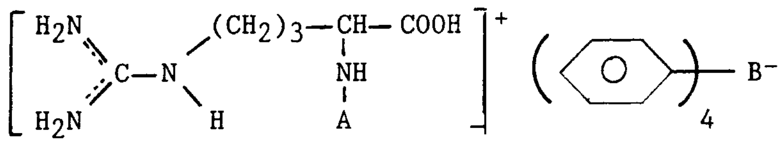

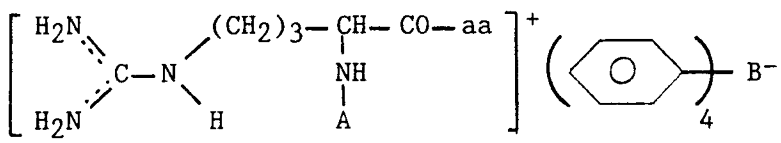

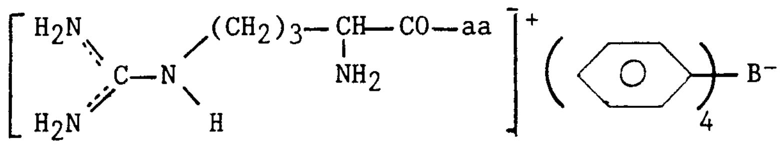

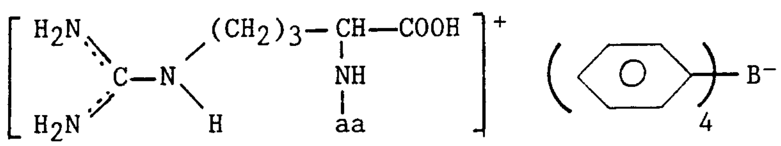

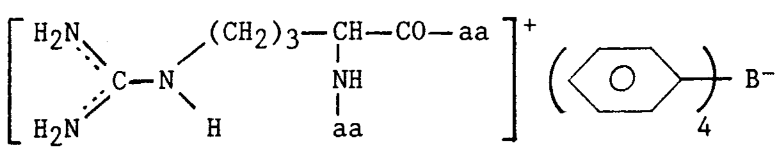

dans lesquelles aa représente un acide aminé ou un peptide lié par une liaison peptidique à l'arginine et dont les fonctions amines et carboxyliques sont éventuellement protégées ou dont la fonction carboxylique terminale est éventuellement en outre substituée par un groupement aminé tel que le groupement $-NH_2$ ou $-NH-CH_2-CH_3$.

4. Procédé pour la préparation des composés selon la revendication 1 caractérisé en ce qu'il met en oeuvre un sel de tétraphénylborate.

5. Procédé selon la revendication 4 caractérisé en ce que le sel de tétraphénylborate mis en oeuvre est formé à partir d'une base organique azotée telle qu'une amine.

6. Procédé selon la revendication 5 caractérisé en ce que l'amine est choisie dans le groupe formé par la triéthylamine, la diisopropyléthylamine, la N-méthylmorpholine, la N-éthylmorpholine, la N-méthylpiperidine, la N-éthylpiperidine, la tri-n-butylamine, la dicyclohexylamine et l'imidazole.

7. Procédé selon l'une quelconque des revendications 4 à 6 caractérisé en ce qu'on met en oeuvre un solvant tel que le N-N diméthylformamide.

8. Utilisation des composés selon l'une quelconque des revendications 1 à 3 lors de la synthèse

peptidique par voie chimique.

**Revendications pour les Etats contractants suivants : ES, GR**

**1.** Procédé pour la préparation de composés guanidiniques comprenant un ion tétraphénylborate répondant à la formule générale :

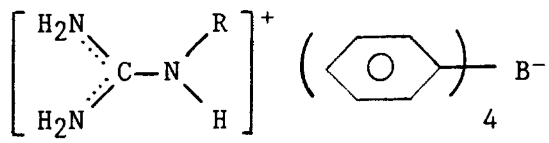

dans laquelle R représente un radical organique de formule générale :

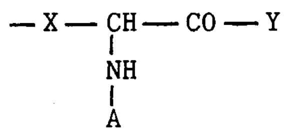

dans laquelle :

- X représente un radical aliphatique linéaire, ramifié ou cyclique, non substitué, saturé ou non, contenant jusqu'à 25 atomes de carbone,
- A représente un atome d'hydrogène, un radical aliphatique ou aromatique comportant des hétéroatomes ou non, tels que le benzyloxycarbonyle ou le tert-butyloxycarbonyle agissant comme groupement protecteur ou le N-hydroxysuccinimide agissant comme groupement d'activation ; un ou plusieurs acides aminés liés par des liaisons peptidiques, dont certaines fonctions sont éventuellement substituées par le benzyloxycarbonyle ou le tert-butyloxycarbonyle agissant comme groupement protecteur ou le N-hydroxysuccinimide agissant comme groupement d'activation, un groupement protecteur tel que le benzyloxycarbonyle ou le ter-butyloxycarbonyle,
- Y représente un groupement hydroxyle ; un atome d'halogène ; un radical aliphatique ou aromatique, comportant éventuellement des hétéroatomes, tels que le benzyloxycarbonyle ou le tert-butyloxycarbonyle agissant comme groupement protecteur ou le N-hydrosuccinimide agissant comme groupement d'activation ; un groupement aminé ; un acide aminé ou un peptide dont certaines fonctions sont éventuellement substituées par des groupements protecteurs ou des groupements d'activation ainsi que par des groupements aminés de formule générale $NR_1R_2$ dans laquelle $R_1$ et $R_2$ représentent indépendamment l'un de l'autre un atome d'hydrogène ou un groupement alkyle comptant de 1 à 3 atomes de carbone,

caractérisé en ce qu'il met en oeuvre un sel de tétraphénylborate et un produit comprenant un groupement guanidinique.

**2.** Procédé selon la revendication 1 caractérisé en ce que :

- X représente le radical $-(CH_2)_3-$ ;
- A représente un atome d'hydrogène, un groupement protecteur tel que le benzyloxycarbonyle ou le tert-butyloxycarbonyle ou un acide aminé éventuellement substitué par le benzyloxycarbonyle, le tert-butyloxycarbonyle ou le N-hydroxysuccinimide.
- Y représente un groupement hydroxyle ou un acide aminé éventuellement substitué par le benzyloxycarbonyle, le tert-butyloxycarbonyle ou le N-hydroxysuccinimide ou un groupement aminé.

**3.** Procédé selon la revendication 1 ou 2 caractérisé en ce que l'on met en oeuvre un sel de tétraphénylborate formé à partir d'une base organique azotée telle qu'une amine.

**4.** Procédé selon la revendication 3 caractérisé en ce que l'on met en oeuvre une amine choisie dans le groupe formé par la triéthylamine, la diisopropyléthylamine, la N-méthylmorpholine, la N-éthylmorpholine, la N-méthylpiperidine, la N-éthylpiperidine, la tri-n-butylamine, la dicyclohexylamine et l'imidazole.

**5.** Procédé selon l'une quelconque des revendications 1 à 4 caractérisé en ce qu'on met en oeuvre un solvant tel que le N-N diméthylformamide.

**6.** Utilisation des composés obtenus selon l'une quelconque des revendications 1 à 5 lors de la synthèse peptidique par voie chimique.

**Claims**
**Claims for the following Contracting States : AT, BE, CH, DE, FR, GB, IT, LI, LU, NL, SE**

**1.** Guanidine-related compounds comprising a tetraphenylborate ion, which are characterised in that they correspond to the general formula:

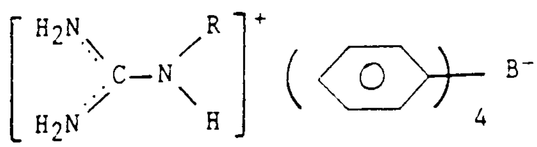

in which R denotes an organic radical of general formula:

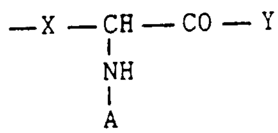

in which:
- X denotes a linear, branched or cyclic, unsubstituted, saturated or unsaturated aliphatic radical containing up to 25 carbon atoms,
- A denotes a hydrogen atom, an aliphatic or aromatic radical containing heteroatoms or otherwise, such as benzyloxycarbonyl or tert-butyloxycarbonyl acting as a protecting group or N-hydroxysuccinimide acting as an activating group, one or more amino acids linked by peptide bonds, in which certain functional groups are optionally substituted by benzyloxycarbonyl or tert-butyloxycarbonyl acting as a protecting group or N-hydroxysuccinimide acting as an activating group, or a protecting group such as benzyloxycarbonyl or tert-butyloxycarbonyl,
- Y denotes a hydroxyl group, a halogen atom or an aliphatic or aromatic radical optionally containing heteroatoms, such as benzyloxycarbonyl or tert-butyloxycarbonyl acting as a protecting group or N-hydrosuccinimide acting as an activating group, an amino group, an amino acid or a peptide in which some functional groups are optionally substituted by protecting groups or activating groups as well as by amino groups of general formula $NR_1R_2$ in which $R_1$ and $R_2$ independently of each other denote a hydrogen atom or an alkyl group containing from 1 to 3 carbon atoms.

**2.** Compounds according to Claim 1, characterised in that:
- X denotes the radical $-(CH_2)_3-$;
- A denotes a hydrogen atom, a protecting group such as benzyloxycarbonyl or tert-butyloxycarbonyl or an amino acid optionally substituted by benzyloxycarbonyl, tert-butyloxycarbonyl or N-hydroxysuccinimide, and
- Y denotes a hydroxyl group or an amino acid optionally substituted by benzyloxycarbonyl, tert-butyloxycarbonyl or N-hydroxysuccinimide or an amino group.

**3.** Compounds according to Claim 1 or 2, characterised in that they correspond to the formulae:

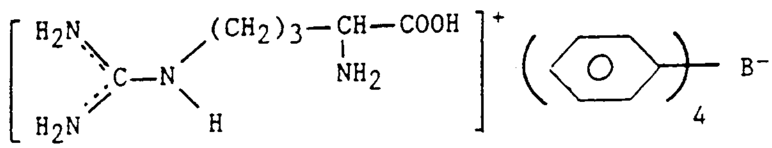

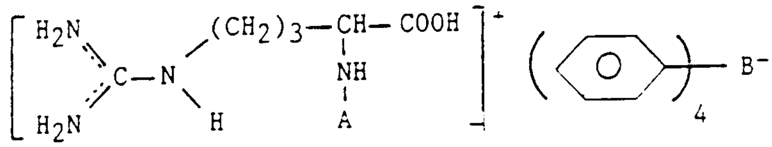

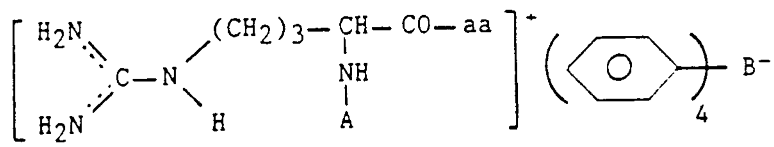

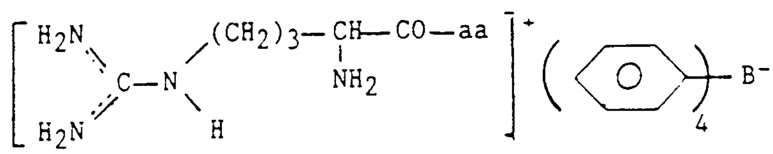

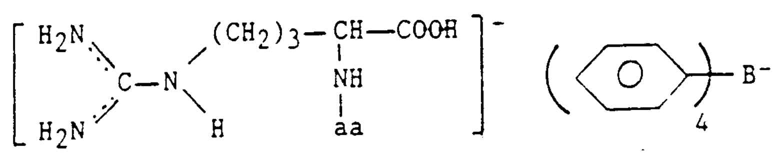

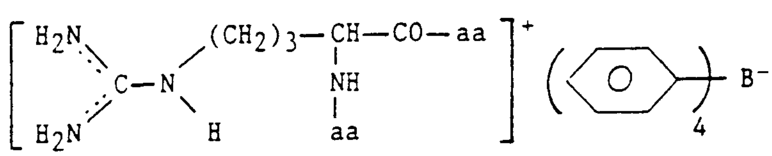

in which aa denotes an amino acid or a peptide linked to arginine by a peptide bond and in which the amine and carboxylic functional groups are optionally protected or whose terminal carboxylic functional group is optionally additionally substituted by an amino group such as the $-NH_2$ or $-NH-CH_2-CH_3$ group.

**4.** Process for the preparation of the compounds according to Claim 1, characterised in that it employs a tetraphenylborate salt.

**5.** Process according to Claim 4, characterised in that the tetraphenylborate salt employed is formed from a nitrogenous organic base such as an amine.

**6.** Process according to Claim 5, characterised in that the amine is chosen from the group consisting of triethylamine, diisopropylethylamine, N-methylmorpholine, N-ethylmorpholine, N-methylpiperidine, N-ethylpiperidine, tri-n-butylamine, dicyclohexylamine and imidazole.

**7.** Process according to any one of Claims 4 to 6, characterised in that a solvent such as N,N-dimethylformamide is employed.

8. Use of the compounds according to any one of Claims 1 to 3 in the synthesis of peptides by a chemical route.

**Claims for the following Contracting States : ES, GR**

1. Process for the preparation of guanidine-related compounds comprising a tetraphenylborate ion corresponding to the general formula:

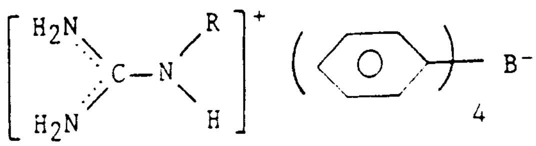

in which R denotes an organic radical of general formula:

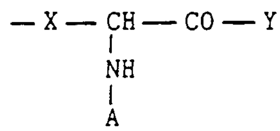

in which:
- X denotes a linear, branched or cyclic, unsubstituted, saturated or unsaturated aliphatic radical containing up to 25 carbon atoms,
- A denotes a hydrogen atom, an aliphatic or aromatic radical containing heteroatoms or otherwise, such as benzyloxycarbonyl or tert-butyloxycarbonyl acting as a protecting group or N-hydroxysuccinimide acting as an activating group, one or more amino acids linked by peptide bonds, in which certain functional groups are optionally substituted by benzyloxycarbonyl or tert-butyloxycarbonyl acting as a protecting group or N-hydroxysuccinimide acting as an activating group, or a protecting group such as benzyloxycarbonyl or tert-butyloxycarbonyl,
- Y denotes a hydroxyl group, a halogen atom or an aliphatic or aromatic radical optionally containing heteroatoms, such as benzyloxycarbonyl or tert-butyloxycarbonyl acting as a protecting group or N-hydrosuccinimide acting as an activating group, an amino group, an amino acid or a peptide in which some functional groups are optionally substituted by protecting groups or activating groups as well as by amino groups of general formula $NR_1R_2$ in which $R_1$ and $R_2$ independently of each other denote a hydrogen atom or an alkyl group containing from 1 to 3 carbon atoms,

characterised in that it makes use of a tetraphenylborate salt and of a product containing a guanidine group.

2. Process according to claim 1, characterised in that:
- X denotes the radical $(CH_2)_3$;
- A denotes a hydrogen atom, a protecting group such as benzyloxycarbonyl or tert-butyloxycarbonyl or an amino acid optionally substituted by benzyloxycarbonyl, tert-butyloxycarbonyl or N-hydroxysuccinimide, and
- Y denotes a hydroxyl group or an amino acid optionally substituted by benzyloxycarbonyl, tert-butyloxycarbonyl or N-hydroxysuccinimide or an amino group.

3. Process according to Claim 1 or 2, characterised in that a tetraphenylborate salt formed from a nitrogenous organic base such as an amine is employed.

4. Process according to Claim 3, characterised in that an amine chosen from the group consisting of triethylamine, diisopropylethylamine, N-methylmorpholine, N-ethylmorpholine, N-methylpiperidine, N-ethylpiperidine, tri-n-butylamine, dicyclohexylamine and imidazole is employed.

5. Process according to any one of Claims 1 to 4, characterised in that a solvent such as N,N-dimethylformamide is employed.

6. Use of the compounds obtained according to any one of Claims 1 to 5 in the synthesis of peptides by a chemical route.

**Patentansprüche**

**Patentansprüche für folgende Vertragsstaaten : AT, BE, CH, DE, FR, GB, IT, LI, LU, NL, SE**

1. Guanidinverbindungen, die ein Tetraphenylboration umfassen, dadurch gekennzeichnet, daß sie der allgemeinen Formel:

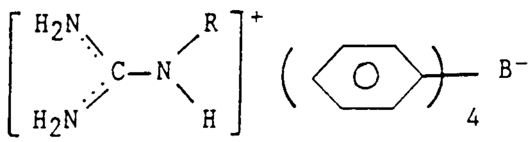

entsprechen, in der R einen organischen Rest der allgemeinen Formel:

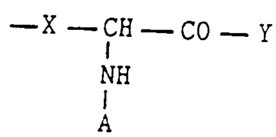

darstellt, in der

X einen linearen, verzweigten oder zyklischen, nicht substituierten, gesättigten oder ungestättigten aliphatischen Rest, der bis zu 25 Kohlenstoffatome enthält, darstellt

A ein Wasserstoffatom, einen aliphatischen oder aromatischen Rest, der Heteroatome oder keine umfaßt, wie das Benzyloxycarbonyl oder tert-Butyloxycarbonyl, die als Schutzgruppierung agieren oder N-Hydroxysuccinimid, das als Aktivierungsgruppierung agiert, eine oder mehrere durch Peptidbindungen verbundene Aminosäuren darstellt, wobei gewisse Funktionen gegebenenfalls substituiert sind durch das Benzyloxycarbonyl oder tert-Butyloxycarbonyl, die als Schutzgruppierung agieren oder N-Hydroxysuccinimid, das als Aktivierungsgruppierung agiert, eine Schutzgruppierung, wie das Benzyloxycarbonyl oder das tert-Butyloxycarbonyl,

Y eine Hydroxylgruppierung, ein Halogenatom, einen aliphatischen oder aromatischen Rest, der gegebenenfalls Heteroatome umfaßt, wie das Benzyloxycarbonyl oder das tert-Butyloxycarbonyl, die als Schutzgruppierung agieren oder das N-Hydroxysuccinimid, das als Aktivierungsgruppierung agiert, eine Amingruppierung, eine Aminosäure oder ein Peptid darstellt, wobei gewisse Funktionen gegebenenfalls durch Schutzgruppierungen oder Aktivierungsgruppierungen ebensowie durch Amingruppierungen der allgemeinen Formel $NR_1R_2$ substituiert sind, in der $R_1$ und $R_2$ unabhänigig voneinander ein Wasserstoffatom oder eine Alkylgruppierung mit 1 bis 3 Kohlenstoffatomen, darstellen.

2. Verbindungen gemäß Anspruch 1, dadurch gekennzeichnet, daß

X den Rest $-(CH_2)_3-$ darstellt;

A ein Wasserstoffatom, eine Schutzgruppierung wie das Benzyloxycarbonyl oder das tert-Butyloxycarbonyl oder eine Aminosäure, die gegebenenfalls durch das Benzyloxycarbonyl, das tert-Butyloxycarbonyl oder das N-Hydroxysuccinimd substituiert ist, darstellt;

Y eine Hydroxylgruppierung oder eine Aminosäure, gegebenenfalls substituiert durch das Benzyloxycarbonyl, das tert-Butyloxycarbonyl oder N-Hydroxysuccinimid oder eine Amingruppierung darstellt.

3. Verbindungen nach Anspruch 1 oder 2, dadurch gekennzeichnet, daß sie den Formeln

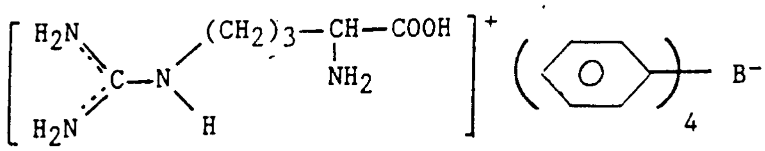

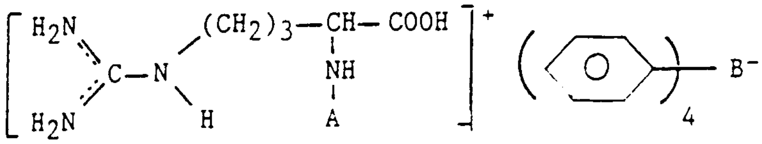

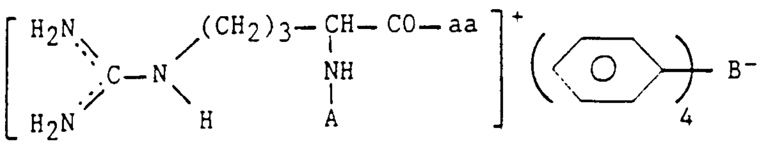

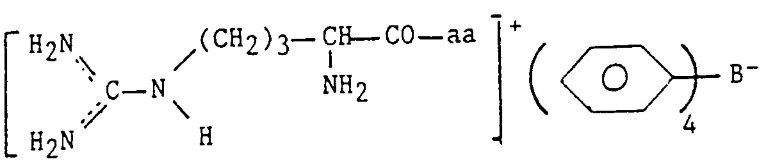

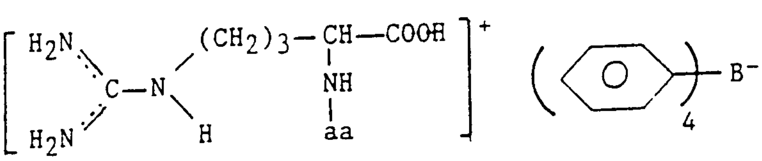

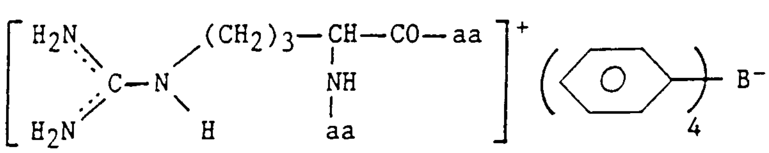

entsprechen, in denen aa eine Aminosäure oder ein durch eine Peptidbindung an Arginin gebundenes Peptid darstellt und deren Amin- und Carboxylfunktionen gegebenenfalls geschützt sind und deren terminale Carboxylfunktion gegebenenfalls außerdem durch eine Amingruppierung, wie die Gruppierung $-NH_2$ oder $-NH-CH_2-CH_3$ substituiert ist.

**4.** Verfahren zur Herstellung der Verbindungen gemäß Anspruch 1, dadurch gekennzeichnet, daß ein Tetraphenylboratsalz eingesetzt wird.

**5.** Verfahren nach Anspruch 4, dadurch gekennzeichnet, daß das verwendete Tetraphenylboratsalz ausgehend von einer stickstoffhaltigen organischen Base, wie einem Amin, gebildet wird.

**6.** Verfahren nach Anspruch 5, dadurch gekennzeichnet, daß das Amin ausgewählt ist aus der Gruppe, gebildet durch das Triethylamin, das Diisopropylethylamin, das N-Methylmorpholin, das N-Ethylmorpholin, das N-Methylpiperidin, das N-Ethylpiperidin, das Tri-n-butylamin, das Dicyclohexylamin und das Imidazol.

**7.** Verfahren nach einem der Ansprüche 4 bis 6, dadurch gekennzeichnet, daß man ein Lösungsmittel, wie N-N-Dimethylformamid einsetzt.

8. Verwendung der Verbindungen nach einem der Ansprüche 1 bis 3 bei der Peptidsynthese auf chemischem Wege.

**Patentansprüche für folgende Vertragsstaaten : ES, GR**

1. Verfahren zur Herstellung von ein Tetraphenylboration umfassenden Guanidinverbindungen, die der allgemeinen Formel:

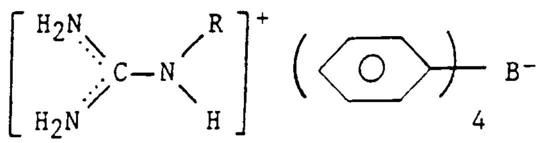

entsprechen, in der R einen organischen Rest der allgemeinen Formel:

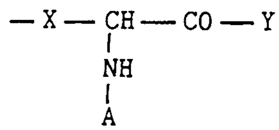

darstellt, in der
   X einen linearen, verzweigten oder zyklischen, nicht substituierten, gesättigten oder ungestättigten aliphatischen Rest, der bis zu 25 Kohlenstoffatome enthält, darstellt
   A ein Wasserstoffatom, einen aliphatischen oder aromatischen Rest, der Heteroatome oder keine umfaßt, wie das Benzyloxycarbonyl oder tert-Butyloxycarbonyl, die als Schutzgruppierung agieren oder das N-Hydroxysuccinimid, das als Aktivierungsgruppierung agiert, eine oder mehrere durch Peptidbindungen verbundene Aminosäuren darstellt, wobei gewisse Funktionen gegebenenfalls substituiert sind durch das Benzyloxycarbonyl oder das tert-Butyloxycarbonyl, die als Schutzgruppierung agieren oder das N-Hydroxysuccinimid, das als Aktivierungsgruppierung agiert, eine Schutzgruppierung, wie das Benzyloxycarbonyl oder das tert-Butyloxycarbonyl,
   Y eine Hydroxylgruppierung, ein Halogenatom, einen aliphatischen oder aromatischen Rest, der gegebenenfalls Heteroatome umfaßt, wie das Benzyloxycarbonyl oder tert-Butyloxycarbonyl, die als Schutzgruppierung agieren, oder das N-Hydroxysuccinimid, das als Aktivierungsgruppierung agiert, eine Amingruppierung, eine Aminosäure oder ein Peptid darstellt, wobei gewisse Funktionen gegebenenfalls substituiert sind durch Schutzgruppierungen oder Aktivierungsgruppierungen ebensowie durch Amingruppierungen der allgemeinen Formel $NR_1R_2$, in der $R_1$ und $R_2$ unabhänigig voneinander ein Wasserstoffatom oder eine Alkylgruppierung mit 1 bis 3 Kohlenstoffatomen, darstellen,

dadurch gekennzeichnet, daß ein Tetraphenylboratsalz und ein eine Guanidingruppierung umfassendes Produkt verwendet werden.

2. Verfahren gemäß Anspruch 1, dadurch gekennzeichnet, daß
   X den Rest $-(CH_2)_3-$ darstellt;
   A ein Wasserstoffatom, eine Schutzgruppierung wie das Benzyloxycarbonyl oder das tert-Butyloxycarbonyl oder eine Aminosäure, die gegebenenfalls durch das Benzyloxycarbonyl, das tert-Butyloxycarbonyl oder das N-Hydroxysuccinimid substituiert ist, darstellt;
   Y eine Hydroxylgruppierung oder eine Aminosäure, gegebenenfalls substituiert durch das Benzyloxycarbonyl, das tert-Butyloxycarbonyl oder N-Hydroxysuccinimid oder eine Amingruppierung darstellt.

3. Verfahren nach Anspruch 1 oder 2, dadurch gekennzeichnet, daß ein Tetraphenylboratsalz, gebildet ausgehend von einer stickstoffhaltigen organischen Base, wie einem Amin, verwendet wird.

4. Verfahren nach Anspruch 3, dadurch gekennzeichnet, daß ein Amin, ausgewählt aus der Gruppe

gebildet durch das Triethylamin, das Diisopropylethylamin, das N-Methylmorpholin, das N-Ethylmorpholin, das N-Methylpiperidin, das N-Ethylpiperidin, das Tri-n-butylamin, das Dicyclohexylamin und das Imidazol, verwendet wird.

**5.** Verfahren nach einem der Ansprüche 1 bis 4, dadurch gekennzeichnet, daß ein Lösungsmittel wie das N-N-Dimethylformamid verwendet wird.

**6.** Verwendung der Verbindungen, erhalten nach einem der Ansprüche 1 bis 5, bei der Peptidsynthese auf chemischem Weg.